(19) **Europäisches Patentamt European Patent Office Office européen des brevets**

(11) **EP 4 238 564 A1**

(12) **EUROPEAN PATENT APPLICATION**

(43) Date of publication:
06.09.2023 Bulletin 2023/36

(21) Application number: 22159838.6

(22) Date of filing: 02.03.2022

(51) International Patent Classification (IPC):
**A61K 31/4045** (2006.01) **A61K 31/506** (2006.01)
**A61K 31/585** (2006.01) **A61P 35/00** (2006.01)

(52) Cooperative Patent Classification (CPC):
(C-Sets available)
**A61P 35/00; A61K 31/4045; A61K 31/505;
A61K 31/506; A61K 31/5377; A61K 31/585;
A61K 45/06; C07D 239/48; C07D 401/12;
C07D 403/12; C07D 413/12; C07D 417/14** (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Bash Biotech Inc**
**San Diego, CA 92101 (US)**

(72) Inventors:
• **LI, Xiangyu**
**113 46 Stockholm (SE)**
• **MARDINOGLU, Adil**
**412 60 Göteborg (SE)**

(74) Representative: **Kransell & Wennborg KB**
**P.O. Box 27834**
**115 93 Stockholm (SE)**

(54) **NEW TREATMENTS OF RENAL CELL CARCINOMA**

(57) There is provided a compound or a pharmaceutically acceptable salt or prodrug thereof for use in a method of treatment of a renal cell carcinoma, wherein the compound is selected from the group consisting of TG-101209, panobinostat, NVP-TAE684 and withaferin-a.

F

**EP 4 238 564 A1**

**(Cont. next page)**

(52) Cooperative Patent Classification (CPC): (Cont.)

C-Sets
**A61K 31/4045, A61K 2300/00;**
**A61K 31/505, A61K 2300/00;**
**A61K 31/506, A61K 2300/00;**
**A61K 31/5377, A61K 2300/00;**
**A61K 31/585, A61K 2300/00**

## Description

## TECHNICAL FIELD

The present disclosure relates to the field of treatment of renal cell carcinoma.

## BACKGROUND

Clear cell renal cell carcinoma (ccRCC) is the most common histological subtype of renal cell carcinoma (RCC), accounts for 70% of all RCC cases [1]. Surgery (radical or partial nephrectomy) is the standard primary treatment for patients with localized tumors. The first-line and second-line target therapy options for patients with relapse after nephrectomy or advanced stage tumor include tyrosine kinase inhibitors (axitinib, sorafenib, pazopanib, and sunitinib, etc.), mTOR inhibitors (everolimus and tesirolimus), and monoclonal antibodies against VEGF, PD-1 or PD-L1 (bevacizumab, pembrolizumab and avelumab, etc.). However, the National Comprehensive Cancer Network (NCCN, version: 1.2022) has reported that the response rates of the single-agent or combinatory regimens based on these drugs range from 6% to 50% in different clinical trials [2]. Moreover, the average duration of disease control with these drugs is only 8-9 months for the first-line setting and 5-6 months for the second-line setting [3]. Therefore, there is a need to discover more tolerated and effective drugs to widen the options for single-agent or combinatory regimens for ccRCC patients.

## SUMMARY

Computational drug repositioning based on systems biology methods has become a powerful tool to identify potential drug-target interactions and drug-disease interactions [4]. The advantage of drug repositioning is that the pharmacology and safety of the repositioned drugs have been well-characterized, dramatically decreasing the cost and duration taken by traditional drug development and reducing the risk of attrition in clinical phases [5,6]. In general, current drug repositioning strategies can be classified into drug-based, disease-based and profile-based [7]. Usually, drug-based and disease-based approaches are conducted by comparing drug-drug or disease-disease similarity or applying existing drug treatment knowledge to predict new disease-drug associations [8,9]. In contrast, profile-based approaches are conducted by analyzing the high-throughput multi-omics data associated with diseases and drugs, which do not rely on prior knowledge about a particular drug or disease and have increased ability to discover new drug-disease pairs [7].

Recently, several studies have employed profile-based repositioning methods to identify potentially valuable drugs for the treatment of ccRCC. A widely used method is selecting the drug that has a reversed effect on the disease signature genes [10,11]. The idea of this method is that if the perturbation of gene expression induced by a drug (drug-perturbed signatures) is negatively correlated with the dysregulation in the tumor tissues compared to normal tissues (disease-specific signatures), this drug turns out to have therapeutic value for this tumor type. During the application of the above approach, ConnectivityMap (CMap) [12] is the most commonly used drug-perturbed gene expression data source, and it has been recently updated and integrated into the LINCS Data Portal [13]. To date, the LINCS data portal includes more than three million gene expression profiles associated with more than 20,000 drugs, gene overexpression and knockdown in up to more than 200 cell lines [13]. However, a drug repurposed in this way is supposed to have multiple gene targets mixed by oncogenes and passenger genes, limiting the identification and validation of the key targets and mechanisms of drug effect.

By starting from the target prediction and then repurposing the existed inhibitor of the target genes for cancer treatment this problem has been avoided. In a previous study, a list of candidate target genes which are essential for the ccRCC tumor cell growth by the genome-scale metabolic model analysis were identified [14]. Among these essential genes, three genes, *SOAT1, CRLS1* and ACACB, whose inhibition is not toxic in the 32 major normal human tissues have been filtered out by performing an *in silico* toxicity test for each essential gene, as the final targetable genes. Finally, a well-known inhibitor of *SOAT1,* mitotane, was repurposed and its drug efficacy for the treatment of ccRCC was validated. However, one limitation of this study is that a systematic drug repositioning approach is needed to discover more potentially effective drugs that could inhibit *SOAT1.* In addition, the list of candidate target genes need to be extended and this extension should not be limited to the metabolically important genes, which covers only around 20-25% of the human genome (as reported in the human metabolic model [15]), but should also include non-metabolic genes. Accordingly, gene co-expression network (GCN) analysis that applies all possible human genes has been used to identify the key genes, its neighbors and functionally related biological functions [5,16].

In the study of the present disclosure, an integrated strategy that involved disease-target prediction and drug-target prediction was applied. First, a set of robust ccRCC signature genes whose expression was significantly associated with patients' survival outcomes were extracted. Functional enrichment analysis showed that these genes were significantly enriched in the cell division, cell cycle, DNA replication, angiogenesis, cell migration and cell differentiation pathways, all of which are well-known hallmarks in cancer [17]. Second, two types of molecular modules that significantly enriched with the unfavorable and favorable signatures were identified based on the GCN analysis of the transcriptomic data of ccRCC tissues. Third, four target genes, i.e.

*BUB1B, RRM2, ASF1B* and *CCNB2,* showing high centrality in the modules were extracted. Next, a drug repositioning approach based on the analysis of shRNA-perturbed and drug-perturbed transcriptomics data from the LINCS data portal was developed and the three most effective drugs for each target were repurposed. Finally, the drug effects were tested in Caki-i cells. These tests showed that TG-101209, Panobinostat, NVP-TAE684 and withaferin-a were the most effective drugs.

Accordingly, the present disclosure provides a compound or a pharmaceutically acceptable salt or prodrug thereof for use in a method of treatment of a renal cell carcinoma, such as clear cell renal cell carcinoma (ccRCC), wherein the compound is selected from the group consisting of TG-101209, panobinostat, NVP-TAE684 and withaferin-a.

**BRIEF DESCRIPTION OF THE DRAWINGS**

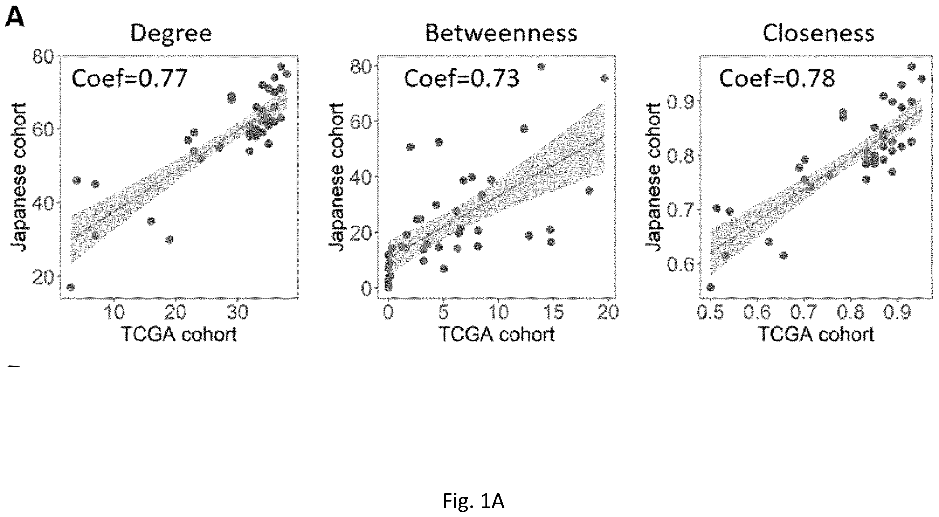

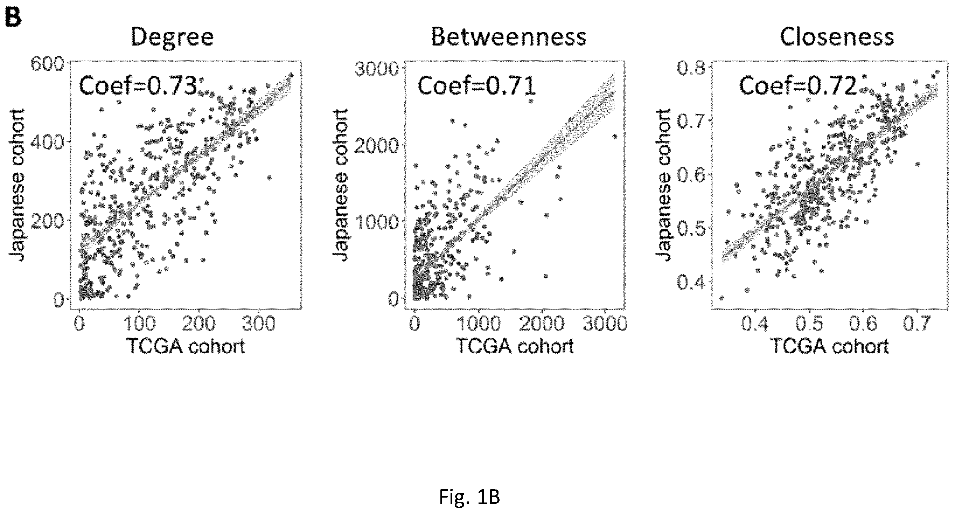

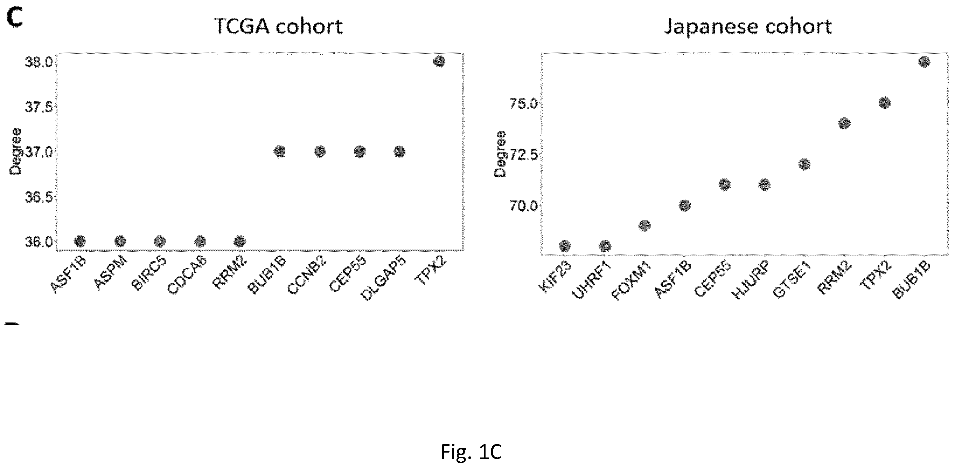

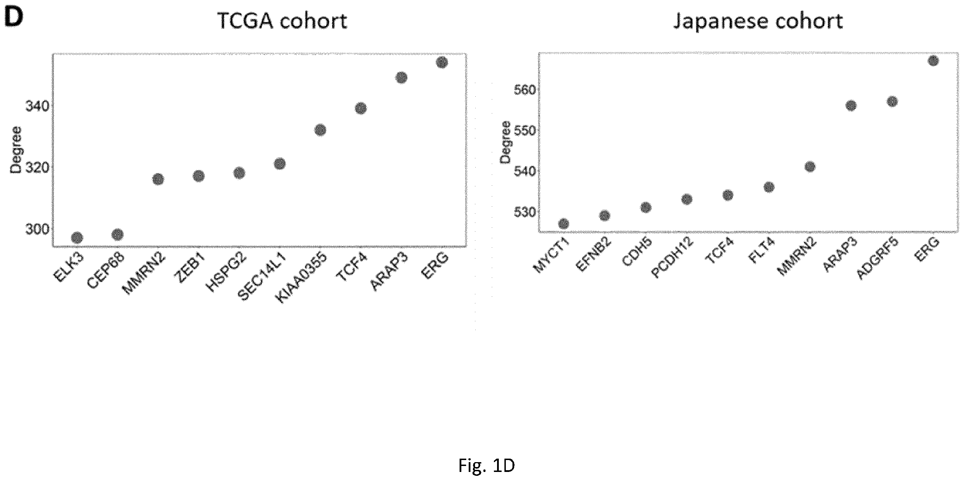

Fig. 1 shows a topology analysis of the unfavorable and favorable modules. A) Spearman correlation of degree, betweenness or closeness centralities of genes from M11 in the TCGA cohort and M3 in the Japanese cohort. Coef, Spearman correlation coefficient. B) Spearman correlation of degree, betweenness, and closeness centralities between genes from the merged module M1/2/3/4/18 in the TCGA cohort and M1 in the Japanese cohort. Coef, the Spearman correlation coefficient. The gray area indicates the 95% confidence interval for the prediction of the linear model. C) The top 10 genes with the highest degree values in M11 from the TCGA cohort and M3 from the Japanese cohort, respectively. The common genes between the two cohorts are ASF1B, RRM2, *BUB1B,* CEP55 and TPX2. D) The top 10 genes with the highest degree centrality in M1/2/3/4/18 from the TCGA cohort and M1 from the Japanese cohort, respectively. The common genes between the two cohorts are MMRN2, TCF4, ARAP3 and ERG.

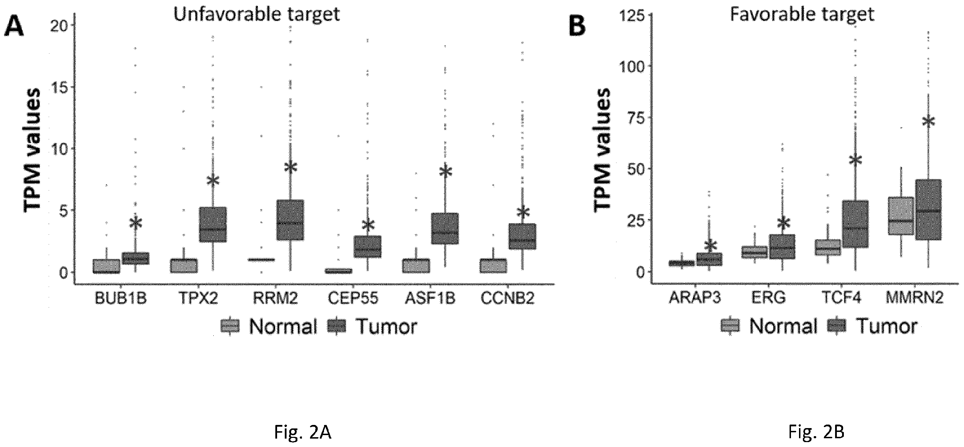

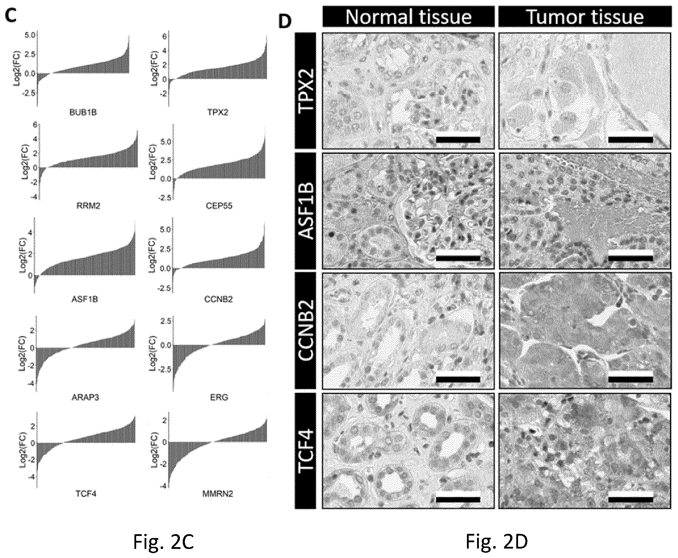

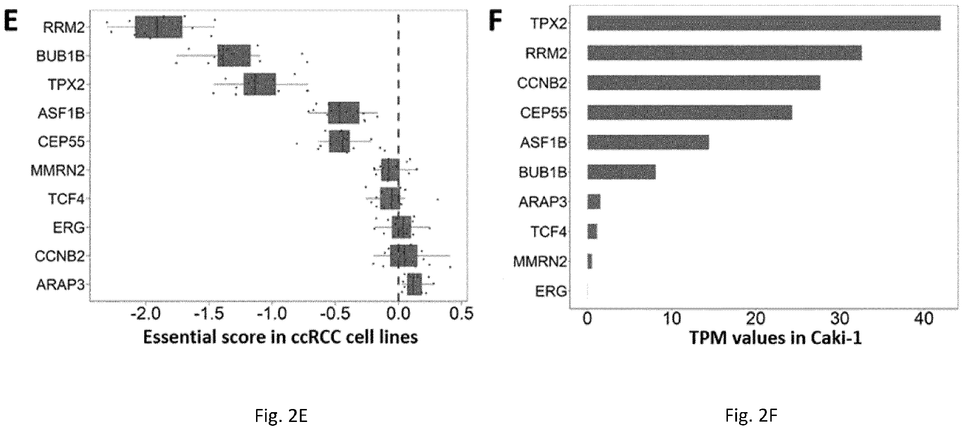

Fig. 2 shows the alteration of hub genes in tumor tissues or cell lines. A) Box plots showing the mRNA expression levels (TPM values) of unfavorable hub genes in TCGA tumors compared to normal tissues. The differential expression analysis was performed by DESeq2. *represents FDR<0.05. B) Box plots showing the mRNA expression levels (TPM values) of favorable hub genes in TCGA tumor tissues compared to normal tissues. *represents FDR<0.05. C) The log2 fold changes between the expression value of a hub gene in each TCGA tumor sample and the average expression value of this gene in TCGA normal samples. The x-axis represents individual tumor samples. Log2(FC), log2 fold change. D) The IHC images of hub genes in normal tissues and tumor tissues. The images related to the same gene were conducted by the same antibody, which was described in the Method section. Scale bar, 50$\mu$m. E) The essential scores of hub genes in 16 ccRCC cell lines. More negative scores indicate more essential for tumor cell survival and proliferation. F) The mRNA expression levels (TPM values) of hub genes in the Caki-i cell line.

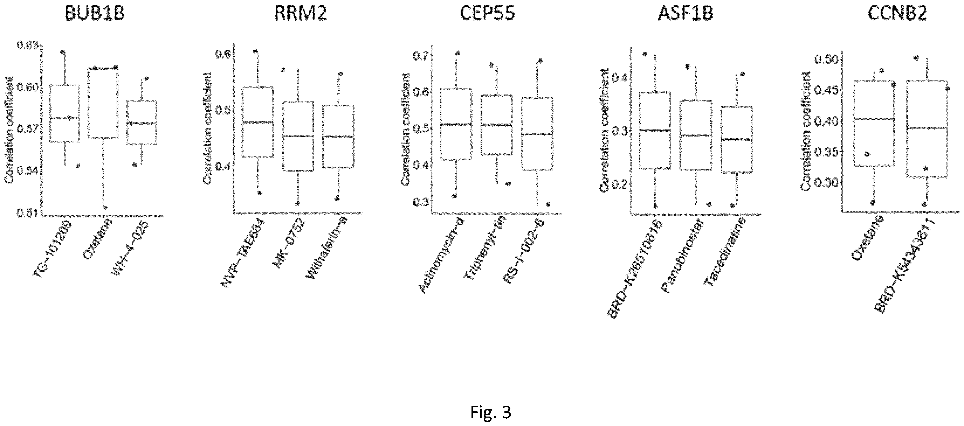

Fig. 3 shows drug repositioning for the target genes. Box plots showing the three most effective drugs for each target gene. Each point represents the similarity (calculated as spearman correlation coefficient) between the repurposed drug perturbed effects and shRNA knockdown perturbed effects on cells. The bottom and top of the boxes represent the 25th and 75th percentiles, respectively. The whiskers represent the minimum and maximum values that are not outliers. The central band represents the median value.

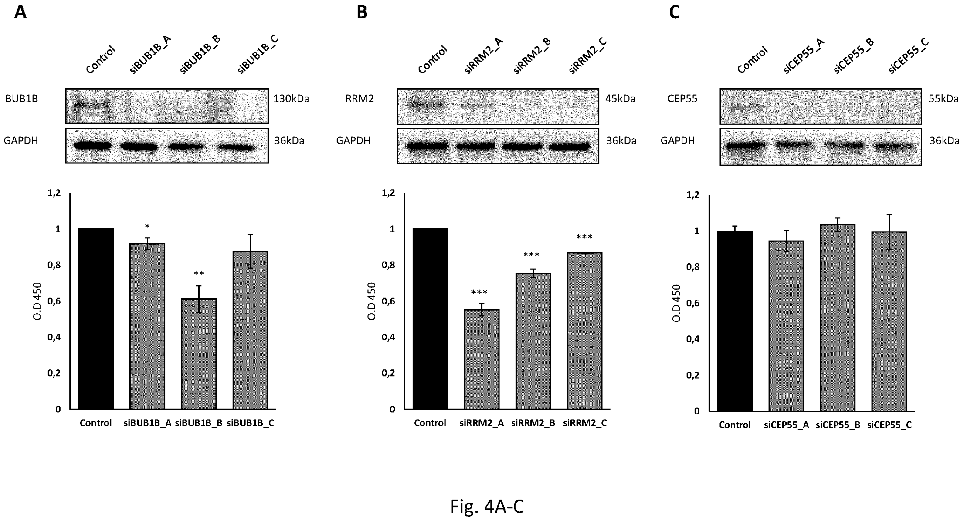

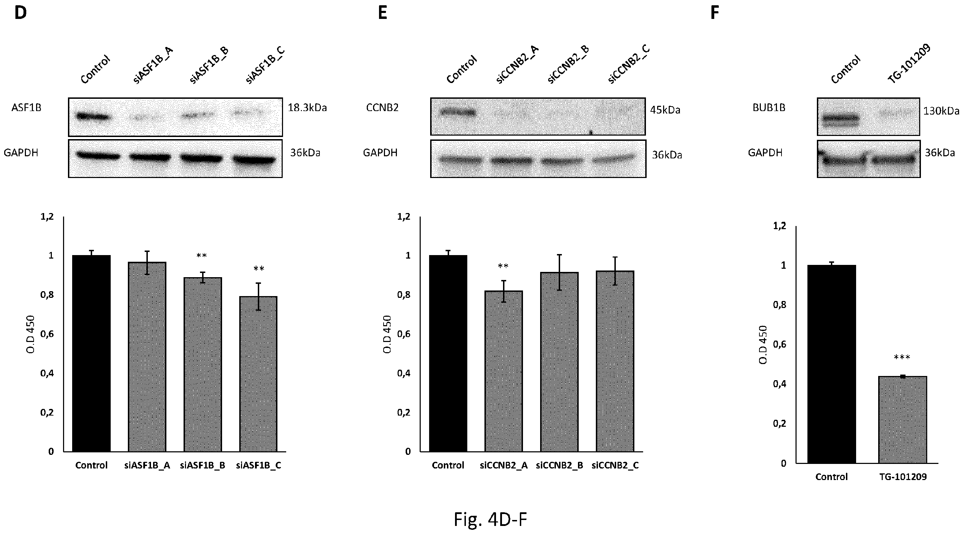

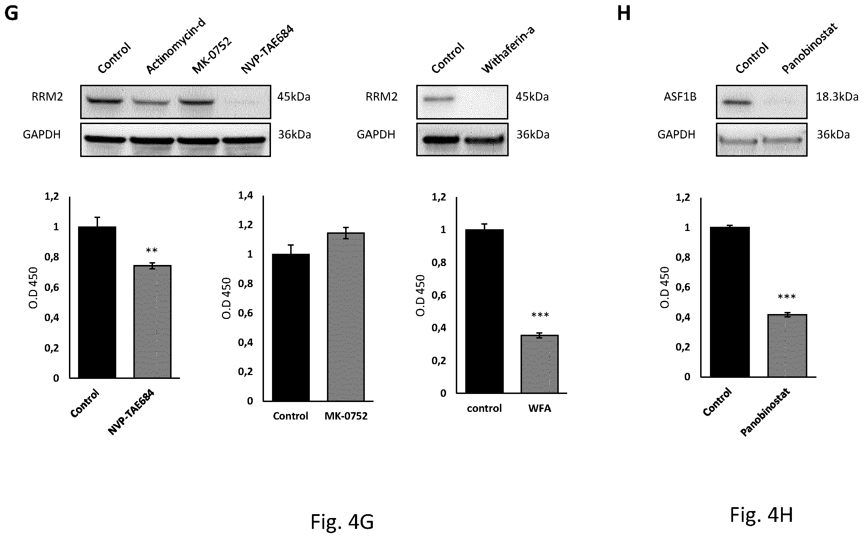

Fig. 4 shows validation of target genes and drug effects. A-E) Western blots showing the protein levels of target genes in Caki-i with siRNA transfection and negative control and bar plots showing the change of cell viability. F-H) Western plots showing that the protein levels of target genes were inhibited by the treatment of their corresponding target drugs and bar plots showing the change of cell viability. The cells in the negative control group were only treated by DMSO. The change of cell viability between the two groups was compared by two-sided T-test. * represents P < 0.05. ** represents P < 0.01, *** represents P < 0.001. Error bars represent standard deviation.

**DETAILED DESCRIPTION**

ccRCC is a heterogeneous tumor that has been stratified into several molecular subtypes characterized by distinct mRNA expression patterns and opposite survival outcomes of patients [14,32,33]. Different subtypes of patients respond differently to chemotherapy. Low response rates and drug resistance exacerbate the challenges in ccRCC therapy. Thus, there is an urgent need for discovering new treatment options for patients who cannot benefit from the commonly used chemotherapies. In the study behind the present disclosure, an integrated approach combining the target prediction and drug repositioning for ccRCC treatment was employed. As a result, four promising druggable target genes (i.e. *BUB1B, RRM2, ASF1B* and *CCNB2),* which encode the proteins involved in cell mitosis and cell cycle regulation were identified for treatment of ccRCC. *BUB1B* encodes a mitotic spindle checkpoint and exhibits a cell cycle dependent expression. It is undetectable in G1 and exhibits a gene expression peak in G2/M [34,35]. *RRM2* encodes ribonucleotide reductase M2 subunit and it is responsible for the ribonucleotide deoxyribonucleotide conversion during the S phase of the cell cycle [36]. Thus, it is only expressed during the late G1 /early S phase and degraded in late S phase [37]. *CCNB2,* encoding cyclin B2, is involved in the G2-M transition in eukaryotes by activating CDC2 kinase [38-40] and it also shows a gene expression peak in G2/M [35]. *ASF1B,* encoding one of the isoforms of the histone $H_3$-$H_4$ chaperone anti-silencing function 1, is necessary for cell proliferation and differently expressed in the cycling and non-cycling cells [41]. It has been reported that *BUB1B* is an independent prognostic marker [42], *RRM2* is a drug resistance-related marker [43], *ASF1B* and *CCNB2* are metastasis markers for ccRCC [44,45]. It is shown that these target genes are significantly upregulated in tumor tissues compared to the adjacent normal tissues. Moreover, broad coverage of upregulated alteration for each of these target genes was observed in ccRCC patients (Figure 2C), implying that most patients may benefit from treatment of these target genes even disease states varied for individual patients.

Further, a drug repositioning approach using the correlation analysis between shRNA-perturbed and drug-perturbed signatures was developed and the three most effective drugs for each target were identified. In the end, the following inhibitory effects were validated using an *in vitro* model:

TG-101209 for targeting *BUB1B;*
NVP-TAE684 and withaferin-a for targeting *RRM2;* and
panobinostat for targeting *ASF1B.*

TG-101209, a JAK2 inhibitor, was originally developed for patients with myeloproliferative disorders who carry the JAK2V617F mutation [46]. It also inhibits the tumor cell growth in myeloma [47] and lung cancer [48] using *in vitro* or *in vivo* models.

NVP-TAE684, an ALK inhibitor, was originally designed to inhibit oncogenic ALK-rearranged fusion proteins (e.g., NPM-ALK) [49]. It has been reported that NVP-TAE684 suppressed the cell proliferation in pancreatic adenocarcinoma [50] and neuroblastoma [51], and reversed multidrug resistance in osteosarcoma [52].

Withaferin-a, a steroidal lactone derived from the medicinal plant *Withania somnifera* Dunal (Solanaceae), has a wide range of pharmacological activities, including cardioprotective, anti-inflammatory, and anti-angiogenesis [53]. It has been reported that withaferin-a induced cell apoptosis by inhibiting the expression or activation of STAT3 in ccRCC cells [54] and other cancer cells [55-57].

Panobinostat, a non-selective histone deacetylase inhibitor, is an FDA-approved oral drug to treat multiple myeloma [58].

Since all the three druggable targets, *BUB1B, RRM2* and *ASF1B,* are cell cycle dependent genes, a combination of two or more of their corresponding repurposed drugs is expected to generate a synergistic effect on the inhibition of the tumor cell growth.

Further, there may be a synergistic effect of combining a repurposed drug with a clinically used first-line chemotherapy drugs (e.g., a tyrosine kinase inhibitor, a mTOR inhibitor or a monoclonal antibody against VEGF).

Accordingly, as a first aspect of the present disclosure, the compound TG-101209 or a pharmaceutically acceptable salt or prodrug thereof is provided for use in a method of treatment of a renal cell carcinoma, such as clear cell renal cell carcinoma (ccRCC). As explained above, TG-101209 targets *BUB1B.*

In an embodiment of the first aspect, the method of treatment further comprises administration of:

i) panobinostat (targeting *ASF1B*); and/or
ii) NVP-TAE684 or withaferin-a (targeting *RRM2).*

As indicated above, such a combination is expected to have a synergistic effect.

As a second aspect of the present disclosure, the compound panobinostat or a pharmaceutically acceptable salt or prodrug thereof is provided for use in a method of treatment of a renal cell carcinoma, such as ccRCC. As explained above, panobinostat targets *ASF1B.*

In a preferred embodiment of the second aspect, the renal cell carcinoma is stage I-III ccRCC, such as stage I or II ccRCC.

In an embodiment of the second aspect, the method of treatment further comprises administration of:

i) TG-101209 (targeting *BUB1B*); and/or
ii) NVP-TAE684 or withaferin-a (targeting *RRM2*).

As indicated above, such a combination is expected to have a synergistic effect.

As a third aspect of the present disclosure, the compound NVP-TAE684 or a pharmaceutically acceptable salt or prodrug thereof is provided for use in a method of treatment of a renal cell carcinoma, such as ccRCC. As explained above, NVP-TAE684 targets *RRM2.*

In an embodiment of the third aspect, the method of treatment further comprises administration of:

i) panobinostat (targeting *ASF1B*); and/or
ii) TG-101209 (targeting *BUB1B*).

As indicated above, such a combination is expected to have a synergistic effect.

As a fourth aspect of the present disclosure, the compound withaferin-a or a pharmaceutically acceptable salt or prodrug thereof is provided for use in a method of treatment of a renal cell carcinoma, such as ccRCC. As explained above, withaferin-a targets *RRM2.*

In an embodiment of the fourth aspect, the method of treatment further comprises administration of:

i) panobinostat (targeting *ASF1B*); and/or
ii) TG-101209 (targeting *BUB1B*).

As indicated above, such a combination is expected to have a synergistic effect.

The subject of the above-mentioned therapeutic methods is preferably a human.

In an embodiment of any one of the above-mentioned aspects, the method of treatment further comprises administration of a drug selected from the group consisting of tyrosine kinase inhibitors (e.g. axitinib, sorafenib, pazopanib and sunitinib), mTOR inhibitors (e.g. everolimus and tesirolimus) and monoclonal antibodies against VEGF (e.g. bevacizumab).

## EXAMPLES - Materials and methods

### Data and preprocessing

Transcript-expression profiles (TPM and count values) of 528 TCGA ccRCC samples and 72 adjacent normal samples were downloaded from https://osf.io/gqrz9[63]. The tumor and normal samples were extracted with sample and vial identifiers of BRC patient barcodes '01A' and '11A', respectively, which represent primary solid tumor tissue and solid normal tissue from the first vial, respectively. The mRNA expression was quantified by Kallisto [64] based on the GENCODE reference transcriptome (version 24) (Ensembl 83 (GRCh38.P5)). The clinical information of TCGA samples was downloaded by using the R package TCGAbiolinks [65]. The mRNA-seq data of 100 ccRCC samples of patients from the Japanese cohort [66] was downloaded from the European Genome-phenome Archive (Accession number: EGAS00001000509). BEDTools[67] was used to convert BAM to FASTQ file, and Kallisto was used for quantifying the count and TPM values of transcripts based on the same reference transcriptome of TCGA data. The sum value of the multiple transcripts of a gene was used as the expression value of this gene. The genes with average TPM values >1 were analyzed.

The essential scores of genes of 16 ccRCC cell lines were downloaded from DepMap Portal (https://depmap.org/portal/) [29], which are estimated based on the CRISPR-Cas9 essentiality screens. The meaning of the score is the essentiality of a gene for cancer cell survival after CRISPR-Cas9 knockout of this gene. More negative scores indicate more essential. The processed RNA-seq data of Caki-i was downloaded from the Cancer Cell Line Encyclopedia (CCLE) portal (version: CCLE_RNAseq_rsem_transcripts_tpm_20180929.txt.gz) [68].

### Survival analysis

Both the univariate Cox regression model and the Kaplan-Meier method were used to evaluate the association of gene expression with patients' overall survival (OS). For Cox analysis, an R package named 'survival' in which the input data is the expression values of each gene (TPM values) across all samples of patients, OS and survival outcomes of patients (death or alive) was used. For Kaplan-Meier analysis, the patients were classified into two groups based on the TPM values of each gene and examined their prognoses. Survival curves were estimated by the Kaplan-Meier method and compared by log-rank test. To choose the best TPM cutoffs for grouping the patients most significantly, all TPM values from the 20$^{th}$ to 80$^{th}$ percentiles used to group the patients, significant differences in the survival outcomes of the two groups were examined, and the value yielding the lowest log-rank P-value was selected.

### Functional enrichment analysis

The enrichGo function of the R package ClusterProfiler was used for Gene Ontology (GO) enrichment [69], which uses the hypergeometric distribution to estimate whether a list of genes is significantly enriched in each GO pathway. False discovery rate (FDR) was adjusted by the Benjamini-Hochberg (BH) method. FDR<0.05 was used to identify significantly enriched pathways.

### GCN analysis and gene module identification

Spearman correlation was used to estimate the correlation between each two genes across all tumor samples. The gene-gene links ranked in the top 1% with the highest correlation coefficients were extracted to construct the co-expression networks. A Random walks-based algorithm, Walktrap [22], was used to capture the gene modules with high transitivity from the co-expression network. The modules with more than 20 nodes and clustering coefficients higher than 0.6 were extracted for further analysis. R package 'igraph' was used for the topology analysis [70].

### Concordance analysis of the prognostic genes

If two lists of prognostic genes, list 1 with $L_1$ genes and list 2 with $L_2$ genes, have k overlapping genes, among which s genes show the exact prognostic directions (both favorable or unfavorable) in the two gene lists, the probability of observing at least s consistent genes by chance can be estimated based on the following cumulative hypergeometric distribution model:

$$P = 1 - \sum_{i=0}^{s-1} \frac{\binom{L_2}{i}\binom{L-L_2}{L_1-i}}{\binom{L}{L_1}}$$

L represents the number of the background genes commonly measured in the datasets from which the prognostic genes are extracted. The two gene lists were considered to be significantly overlapped if P < 0.05. The concordance score $s/k$ is used to represent the consistency between the two lists of genes. The score ranges from o to 1, and the higher concordance score indicates the better consistency of two lists of genes.

The hypergeometric distribution model was also used to test whether the favorable or unfavorable signatures significantly overlap with the genes involved in a functional module.

## Differential expression analysis

DESeq2 [71] was used to identify differentially expressed genes (DEGs) between TCGA tumor tissues and normal tissues groups. The lowly expressed genes with average TPM≤1 were removed, and the raw count values of the remaining genes were used as the input of DESeq2. FDR was adjusted by the BH method. FDR<0.05 was used to identify significant DEGs.

## Drug repositioning

The shRNA- and drug-perturbed signature profiles (level 5 data) in HAIE, a kidney cell, were downloaded from the CMap data portal (https://clue.io/,version: CMAP LINCS 2020) [12]. Three or more biological replicates typically do the experiments in CMap. The level 5 data provides the replicate-collapsed Z-scores, representing a consensus biological response of transcriptomics to the perturbation of drug treatment or shRNA infection derived from different replicates[30]. Totally, 37,669 drug-perturbed signature profiles related to 6,986 drugs with different doses and time points and 21 shRNA-perturbed signature profiles related to *BUB1B* (six shRNAs), *RRM2* (three shRNAs), *CEP55* (three shRNAs), *ASF1B* (three shRNAs) and *CCNB2* (six shRNAs) were obtained.

The drug repositioning approach hypothesizes that a drug is considered to have an inhibitory effect on the expression of a target gene if this drug leads to a wide perturbation on the gene expression landscape in tumor cells, similar to the effect of the knockdown of the target gene. Four procedures were applied to identify the drugs which had the highest possibility to inhibit the expression of their corresponding target genes by an integrated analysis of the shRNA- and drug-perturbed signature profiles. 1) Constructing the drug-shRNA matrix for each target gene. For a given target gene, the shRNA-perturbed signature profiles in HAIE cell line in which the Z-scores represent the biological dysregulation of gene expression in cells after shRNA infection was extracted. As one gene was knocked down by at least three shRNAs in the experiment setting in CMap, the shRNA-perturbed signature profiles were presented as a gene-shRNA matrix. Also, the drug-perturbed signature profiles in HAIE cell line in which the Z-scores represent the biological dysregulation of gene expression in cells after drug treatment were extracted and a gene-drug matrix was thus generated. Then, the Spearman correlation between each two possible lists of drug-perturbed and shRNA-perturbed signatures was calculated and a correlation coefficient matrix, named drug-shRNA matrix, in which each row represents a drug treatment by a specific dose and time point, and each column represents a specific shRNA for the knockdown of this target gene was thus generated. A correlation coefficient in the drug-shRNA matrix represents the similarity of effects on gene expression between specific drug treatment and specific shRNA knockdown. 2) Optimizing the drug-shRNA matrix. Since the drug treatment with different doses and time points could induce different effects on gene expression in cells as well as different similarities with the effects induced by shRNA knockdown, only the optimal dose and time point for each drug whose perturbation showed the highest similarity (correlation coefficient) with shRNA infection was extracted. Thus, the rows were simplified by keeping a unique and optimal dose and time point for each drug in the drug-shRNA matrix. A cell line may respond differently to different shRNAs even though they target the same genes. Thus, the column was simplified by extracting the optimal shRNAs whose signatures consistently correlate with drug perturbed signatures in the drug-shRNA matrix. A clustering analysis of different shRNAs was performed based on the correlation of different columns in the drug-shRNA matrix and the shRNAs, which were clustered together and showed better similarity with drug-perturbed effects, were extracted. Finally, three shRNAs for BUBIB, two shRNAs for RRM2, two shRNAs for CEP55, two shRNAs for ASF1B, and four shRNAs for CCNB2 were extracted. 3) Extracting the top 1% drug candidates. Based on the optimized drug-shRNA matrix, the drugs associated with each shRNA based on the correlation coefficient were ranked from the largest to smallest. A higher rank indicated a higher correlation. A list of drugs involved in the top 1% with the highest ranks associated with each shRNA was first extracted, and then the overlapped drugs among the top 1% lists related to different shRNAs were selected as candidates. 4) Selecting the three most effective drugs for each target. The top three drugs with the highest median ranks were finally considered the most effective drugs for each target.

The drug repositioning for *BUB1B* was taken as an example. The drug-shRNA matrix of *BUB1B* included 37,669 rows associated with 6,986 drugs with different doses and time points and six columns related to six different shRNAs. After optimization, a simplified drug-matrix with 6,986 drugs, each with the best dose and time point and three optimal shRNAs for knockdown of *BUB1B*, was obtained. Then, the top 1% drugs (70 drugs) with the highest correlation coefficients associated with each shRNA were extracted. 24 overlapped drugs were found among the three lists of top 1% drugs. Among these drugs, the top three drugs, TG-101209, oxetane, and WH-4-025, with the highest median ranks, were finally selected as the most effective drugs for targeting *BUB1B*.

## IHC image

The IHC images of ccRCC tumor tissues and normal kidney tissues were downloaded from the Human Protein Atlas website (https://www.proteinatlas.org/)[27,28]. The protocol of IHC was provided in[27,28]. For a given gene, the IHC images of normal tissue and tumor tissue conducted by the same antibody were selected. *TPX2, ASF1B, CCNB2* and *TCF4* IHC images were done by the HPA005487, HPA069385, HPA008873 and HPA020722 antibodies, respectively.

## Cell culture

Human ccRCC cell line Caki-i was obtained from the Karolinska Institute of Environmental Medicine, Stockholm, Sweden, derived from a male ccRCC patient. The cells were cultured in RPMI 1640 medium with 10% fetal bovine serum (FBS) and 1% penicillin/streptomycin. The cells were cultivated at 37°C in a humidified incubator with 5% $CO_2$.

## siRNA transfection

$20 \times 10^3$ cells were plated by quadruplicate into a 96-well plate per well. Day after seeding, 1 pmol siRNAs for targeting *BUB1B, RRM2, ASF1B, CCNB2* and *CEP55* (OriGene Technologies Inc., USA) were transfected into cells using the Lipofectamine® RNAiMAX reagent (Invitrogen) for three days. Then, the cells were used for cell viability assay and protein lysate was harvested for Western blot analysis after 3-day transfection.

## Drug treatment

The drugs, TG-101209, NVP-TAE684, MK-0752, actinomycin-d, and panobinostat were purchased from Selleckchem (S2692, S1108, S2660, S8964, S1030, Selleckchem, Houston, TX, USA), and withaferin-a was purchased from Sigma (W4394, Sigma-Aldrich, Saint Louis, MO, USA). These drugs were dissolved in DMSO. The cells were seeded in a 96-well plate at $20 \times 10^3$ cells per well. Day after seeding, drugs were added to the wells at a proper concentration, TG-101209 (6nM), NVP-TAE684 (3nM), MK-0752 (5nM), withaferin-a (4uM), actinomycin-d (0.3nM), and panobinostat (5nM) and treated for one day. The cells in the negative control group were only treated by DMSO for one day.

## Western blots

The cells were washed with PBS and lysed with CelLytic M (C2978, Sigma-Aldrich, Saint Louis, MO, USA) lysis buffer containing protease inhibitors. The lysates were centrifuged at 12,000 rpm for 5 min, and the supernatant was collected. The proteins were separated by Mini-PROTEAN® TGXTM Precast Gels (Bio-Rad, Berkeley, CA, USA) and transferred to a Trans-Blot Turbo Mini o.2um PVDF Transfer Packs membrane (Bio-Rad, Berkeley, CA, USA) by using Trans-Blot® TurboTM Transfer System (Bio-Rad, Berkeley, CA, USA). The antibodies for BUB1B (HPA008419), RRM2 (HPA056994), $CEP_{55}$ (HPA023430), ASF1B(HPA069385), CCNB2 (HPA008873), and GAPDH (sc47724, Santa Cruz Biotechnology, Inc.) were used for primary immunoblotting. All the antibodies were diluted at 1:10000 concentration. The membranes were incubated in primary antibody solution overnight at 4°C with gentle rocking. Secondary antibody, goat Anti-Rabbit HRP (ab205718) or goat anti-mouse IgG-HRP (sc2005, Santa Cruz Biotechnology, Inc.), was blotted for 30 min at 4°C with gentle rocking. The protein bands were detected with ImageQuanattm LAS 500 (29-0050-63, GE) automatic exposure procedure or 6 min exposure.

## Cell viability assay

Cell proliferation was detected by Cell Counting Kit-8 (CCK-8) assay. The 10 ul of CCK-8 reagent (1:10) was added to each well of 96-well plate with siRNA transfected cells or drug-treated cells by manufacturer's instruction. The 96-well plate was incubated at 37°C for 2 hours and then measured absorbance at 450 nm using a microplate reader (Hidex Sense Beta Plus).

## EXAMPLES - Results

## Identification of ccRCC signature genes

Both Kaplan-Meier analysis and univariate Cox model were applied to investigate the associations of the mRNA expression levels of genes with the patients' OS in TCGA and Japanese ccRCC cohorts. A Kaplan-Meier survival analysis was performed by classifying the patients into two groups with high and low expression (TPM values) of the investigated gene by selecting an optimal cut off from the 20th and 80th expression percentiles yielding the lowest log-rank P-value as in our previous study[18,19]. Meanwhile, the univariate Cox analysis was performed by calculating the hazard ratio of each gene. As a result, an overlap of 7,813 prognostic genes was identified by Kaplan-Meier and Cox survival analysis in the TCGA cohort (FDR<0.05). Similarly, 1,335 prognostic genes were identified in the Japanese cohort (FDR<0.05). The two sets of genes had a significant overlap (n=1,129, hypergeometric distribution test, P < 1.11e-16), and the concordance score of these overlapped genes (both favorable or unfavorable genes) is 99.91%. Among these 1,129 prognostic genes, there were 342 unfavorable genes and 787 favorable genes whose high expression indicated poor and good survival outcomes of patients, respectively. Functional enrichment analysis showed that the unfavorable genes were significantly enriched in cell division and cell cycle pathways. In contrast, the favorable genes were significantly enriched in the angiogenesis, vasculogenesis, cell migration and cell differentiation pathways (FDR<0.05), which are well-known hallmarks in cancer[17]. Therefore, these genes were used as unfavorable and favorable signature genes for ccRCC.

## Identification of functional modules in the GCN

GCN analysis is a powerful method to identify the hub genes that drive the key cellular signaling pathways, including a set of co-expressed or functionally associated genes during tumor development. The conservative co-expressed pattern may confer a selective advantage for tumor cells if it could be validated in independent datasets [20,21]. The Spearman correlation between two possible gene pairs based on the mRNA expression profiles of ccRCC patients in TCGA and Japanese cohort, respectively, was calculated. To balance the sensitivity for detecting the functional gene modules and the robustness for validation in independent datasets, gene-gene links involved in the top 1% with the highest correlation coefficients were extracted to build the GCN. Around 900,000 gene-gene links with correlation coefficients ranging from 0.74 to 1 were obtained in the co-expression network of the TCGA cohort. Then, a random walks-based algorithm, Walktrap[22], was used to identify the gene modules with high transitivity based on the topology of the GCN. To discover functionally meaningful modules in which genes show a good connectivity, the modules with more than 20 genes and clustering coefficients higher than 0.6 were extracted. Finally, 18 modules (M1-18) were obtained in the TCGA cohort. Further, the association of each module with the unfavorable and favorable signature genes was investigated by concordance analysis. It was found that the 41 genes involved in M11 had a significant overlap with the 342 unfavorable signature genes (n=40, hypergeometric test, FDR<0.05). Thus, the M11 was determined as an unfavorable module. Meanwhile, it was observed that the genes involved in M1 (184 genes), M2 (177 genes), M3 (88 genes), M4 (76 genes) and M18 (22 genes) had significant overlaps with the 787 favorable signature genes, respectively (n=51, 86, 33,18 and 6, hypergeometric test, FDR<0.05), which were determined as favorable modules. Then, the biological function of these modules was investigated by functional enrichment analysis. It was found that the genes involved in unfavorable module were significantly enriched in cell division, cell cycle, DNA replication and MAPK activity pathways (FDR<0.05). The genes involved in favorable modules were significantly enriched in the angiogenesis, vasculogenesis, cell migration, cell differentiation, cell adhesion and several signaling pathways (cAMP-mediated signaling, ERK1 and ERK2 cascade, Ras protein signal transduction and Rho protein signal transduction) (FDR<0.05).

Based on the same methodology, around 900,000 gene-gene links with correlation coefficients ranging from 0.68 to 1 in the GCN of the Japanese cohort were obtained. Five modules (M1-5) were found based on the exact cutoff setting. M3 (81 genes) and M1 (770 genes) had significant overlaps with the unfavorable and favorable signature genes, respectively (n=76 and 222, hypergeometric test, FDR<0.05), which were correspondingly determined as unfavorable and favorable modules. Functional enrichment analysis of the two modules exhibited an identical set of biological pathways resulting from the TCGA cohort. Based on these results, it was inferred that M11 from the TCGA cohort and M3 from the Japanese cohort indicated the same unfavorable gene module and M1, M2, M3, M4 and M18 from the TCGA cohort and M1 from the Japanese cohort indicated the same favorable gene module. The individual genes in these two modules were visualized. The M3 from the Japanese cohort wholly covered all the genes involved M11 from the TCGA cohort, which is highly significant overlap (n=41, hypergeometric test, P < 1.1e-16). Moreover, each of M1, M2, M3, M4 and M18 from the TCGA cohort had a significant overlap with M1 from the Japanese cohort (n=98, 168, 78, 73 and 20, hypergeometric, all P < 1.1e-16). Thus, the genes from M1, M2, M3, M4 and M18 were merged in the TCGA cohort and generated an integrated module M1/2/3/4/18. These findings suggested that two types of gene modules that lead to unfavorable and favorable survival outcomes of patients in ccRCC were found, which had high confidence since these modules were validated in an independent cohort with different racial and geographical characteristics.

It was found the genes involved in M7 and M17 from the TCGA cohort were significantly enriched in the innate immune response pathways (phagocytosis, macrophage activation and Toll-like receptor signaling pathway) and the genes involved in M5, M6, M12 and M15 were significantly enriched in the adaptive immune response pathways (T/B cell activation, T cell differentiation and B cell receptor signaling pathway). Interestingly, the genes involved in M2 from the Japanese cohort were significantly enriched in an identical set of both innate and adaptive immune response pathways. Concordance analysis showed that each of the modules from the TCGA cohort mentioned above had a significant overlap with M2 from the Japanese cohort, suggesting these immune-related modules identified from the TCGA cohort were also independently validated in the Japanese cohort. In addition, the genes involved in M4 from the Japanese cohort were enriched in the fatty acid metabolic process. A previous study reports three molecular subtypes of ccRCC characterized by the different activity of energy metabolism in terms of cell viability and proliferation, cell differentiation and fatty acid metabolism[14], which are consistent with the functions of the modules identified in this study. The genes involved in M5 from the Japanese cohort were significantly enriched in the translational initiation, protein targeting to the endoplasmic reticulum, protein targeting to membrane, viral transcription and viral gene expression pathways. Interestingly, two of the three subtypes identified in the previous study were characterized by the opposite dysregulation of these pathways, corresponding to patients' best and poorest survival outcomes[14].

## Identification of the potentially druggable target genes

Analysis of network topology could provide insight into the importance of a given gene in the network. The genes with high centrality are considered as hub genes in the network, and it was predicted that these are potential targets that are worthwhile to be further evaluated. The degree, betweenness and closeness of each gene in each unfavourable and favourable module were calculated. These three measurements are commonly used to characterize the centrality of nodes from three distinct perspectives: a higher degree indicates that the node is involved in more interactions; a higher betweenness indicates that the node acts as a bridge which lies on the shortest path between other nodes; a higher closeness indicates that the node shows shorter paths to all the other nodes and is likely to be the geometric center of the module [23-26]. The Spearman correlation of each centrality measurement between the two unfavorable modules (or favorable modules) identified from TCGA and Japanese datasets were then calculated. As shown in Figures 1A and 1B, the correlation coefficients of degree, betweenness and closeness were 0.77, 0.73 and 0.78 between M11 from the TCGA cohort and M3 from the Japanese cohort, and 0.73, 0.71 and 0.72 between M1/2/3/4/18 from TCGA cohort and M1 from the Japanese cohort. This result suggests that the topology of the unfavorable and favorable modules identified from the TCGA cohort are highly similar to the Japanese cohort even though the two cohorts have different racial and geographical characteristics. The genes involved in each unfavourable and favourable module were ranked based on decreasing order of the degree, betweenness and closeness values. Then the common genes involved in the top 10 gene lists with the highest rank were selected based on at least one of the three centrality measurements in both TCGA and Japanese cohorts as the hub genes. Consequently, six hub genes, namely BUB1B, TPX2, RRM2, CEP55, ASF1B and CCNB2, from the two unfavorable modules (Figure 1C) and four hub genes, including ERG, ARAP3, TCF4 and MMRN2, from the two favorable modules (Figures 1D) were found. Here CD93 was ignored in the favorable modules since it had no significant association with prognoses of patients. The prognostic effects of these hub genes have been established (data not shown).

The expression levels of the 10 hub genes were visualized based on the gene expression profiles of TCGA tumors and adjacent normal samples. As shown in Figures 2A and 2B, most of these hub genes, especially the six unfavorable hub genes, presented very low expression in normal tissues, while their expression levels were significantly increased in the tumor tissues. As these unfavorable hub genes encode cell mitosis and cell cycle-related proteins, this alteration may indicate that low expression of these genes maintains normal cell renewal and metabolism, but increased expression promotes tumorigenesis or tumor progression. Meanwhile, the significantly increased expression levels of favorable hub genes in tumor tissue may imply activating a protective mechanism to mediate anti-tumor effect in cancer. Further, the altered coverage of these hub genes was investigated in TCGA tumor tissues. The result showed 84-96% of patients presented a high expression of unfavorable hub genes, which leads to poor survival outcomes of patients and 59-73% of patients showed an increased expression of favorable genes, which leads to good survival of patients (Figure 2C), suggesting that most of the ccRCC patients may carry the common molecular alterations induced by the unfavorable hub genes but not for their anti-tumor mechanisms. To confirm the expression changes at the protein level, the expression differences observed at the mRNA level were validated using the immunohistochemistry (IHC) images of the hub genes in normal tissue and tumor tissue from the Human Protein Atlas [27,28] (Figure 2D). Four hub genes with selected antibodies showed a higher protein expression in tumor cells than corresponding normal tissues. For example, TPX2 was not detected in normal glomeruli cells, and lowly expressed in normal tubule cell, but showed medium expression in tumor cells (Figure 2D).

Moreover, the essential scores of these hub genes in 16 ccRCC cell lines were extracted from the Dependency Map (DepMap) portal[29]. The essential scores were evaluated based on genome-scale CRISPR-Cas9 loss-of-function screens and corrected by a computational method CERES[29]. A more negative score of a gene indicates this gene is more essential for tumor cell proliferation and survival. As shown in Figure 2E, most of the unfavorable hub genes were more essential than the favorable genes in ccRCC. The mRNA expression levels of these hub genes were also investigated in Caki-i, a commonly used ccRCC cell model. The result showed that the unfavorable hub genes had appropriate expression levels in Caki-i (Figure 2F). Considering these, the unfavorable hub genes are considered more appropriate target genes, and the inhibitory effect on these genes (e.g., shRNAs and chemical inhibitors) should be further evaluated.

## Drug repositioning for target genes

To find the potential therapeutic drugs for candidate targets, a drug repositioning approach was developed based on an integrated analysis of the drug- and shRNA-perturbed signature profiles from the CMap database[30]. It was hypothesized that a drug has an inhibited effect on the expression of a target gene in tumor cells if the drug treatment leads to a similar dysregulation of gene expression induced by shRNA knockdown. In brief, the drug repositioning approach consists of the following four steps (see Method section for details): 1) Constructing the drug-shRNA matrix for each target gene. The drug-perturbed and shRNA-perturbed signature matrix of the HA1E cell line, the only kidney cell line in CMap database, was extracted. *TPX2* was not analyzed in the following analysis because its shRNA knockdown data was not provided in CMap. For each of the other five target genes, a correlation matrix, namely drug-shRNA matrix, was constructed by calculating Spearman correlation between all possible combinations of drug-perturbed and target-specific shRNA-perturbed signatures. The correlation coefficients in this matrix represent the similarity of effects on gene expression induced by specific drug treatment and specific shRNA knockdown. 2) Optimizing the drug-shRNA matrix. For a specific drug, different doses and treated time points were set for cell line treatment in the CMap experiments. Thus, several perturbed profiles associated with the same drug treatment in a cell line were usually obtained. It has been reported that the effects of drug on human cells is highly dependent on the dose setting [31]. Under different doses, drug-target binding affinities could be changed and the downstream pathways can be affected differently, especially for cell cycle related pathways [31]. To maximally represent the drug efficacy, only the optimal dose and time point with the highest similarity with each shRNA knockdown was kept. For a specific gene knockdown experiment, different shRNAs were set for targeting the same gene. These shRNAs are the specific sub-sequences from the investigated gene, whose sequences could be completely different or share some common bases. To maximally represent the gene knockdown efficacy and avoid the effects from the off-target shRNAs, at least two shRNAs which showed a consistently higher correlation with drug treatment were extracted by a clustering analysis. As a result, five simplified drug-shRNA matrixes were obtained and each of the matrixes had 6,986 drugs (rows) while three shRNAs (columns) for *BUB1B,* two shRNA for *RRM2,* two shRNA for *CEP55,* two shRNA for *ASF1B,* and four shRNA for *CCNB2,* respectively. 3) Extracting the top 1% drug candidates. In a given drug-shRNA matrix, the drugs were ranked based on the correlation with each shRNA. The overlapped drugs, which ranked in the top 1% (70) drug lists with the highest correlation with each shRNA as candidates were extracted. 4) Selecting the three most effective drugs for each target. The top three drugs with the highest median rank across different shRNAs were finally considered as the most effective drugs for each target. As shown in Figure 3, it was discovered that TG-101209, oxetane, WH-4-025 target *BUB1B,* NVP-TAE684, MK-0752, and withaferin-a target *RRM2,* actinomycin-d, triphenyl-tin, RS-I-002-6 target *CEP55,* BRD-K26510616, panobinostat and tacedinaline target *ASF1B* and oxetane and BRD-K54343811 target *CCNB2.*

**Validation of target genes and drug effect**

To confirm the essentiality of the target genes in ccRCC, the expression of target genes was inhibited by transfecting siRNAs to Caki-i and investigated the effect on cell viability. Western blots showed that the protein expression of the five target genes was successfully suppressed in Caki-i transfected with siRNAs compared with negative control (Figure 4A-E). A CCK-8 assay was performed to measure cell proliferation. The result indicated that the cell viability was significantly decreased by the knockdown of *BUB1B, RRM2, ASF1B* and *CCNB2* in Caki-i but not for the knockdown of *CEP55* (Figure 4A-E). Thus, *CEP55* was excluded in the following experiments.

Further, it was investigated whether the repurposed drugs could inhibit their corresponding target genes. Only TG-101209, NVP-TAE684, MK-0752, withaferin-a, actinomycin-d, and panobinostat were successfully purchased, and these drugs were tested in Caki-i in the following experiments. As shown in Figure 4F, it was observed that the protein level of *BUB1B* was significantly decreased by the treatment of TG-101209, which was identified as the most effective drug for *BUB1B* compared to the negative control, and the cell viability was also significantly reduced. NVP-TAE684, MK-0752, and withaferin-a were the top three effective drugs that were identified for targeting *RRM2.* As shown in Figure 4G, it was observed that the protein level of RRM2 was significantly suppressed by both NVA-TAE684 and withaferin-a, but not by MK-0752. The cell viability was significantly reduced by NVA-TAE684 and withaferin-a but not by MK-0752, consistent with the result showing in Western blots. The effect of actinomycin-d, actually targeting *CEP55* based on our prediction, on RRM2 was also tested. The Western blot showed that this drug does not work for inhibiting *RRM2.* The protein level of *ASF1B* was significantly decreased by its target drug panobinostat and cell viability was also significantly reduced after the drug treatment (Figure 4H). Unfortunately, the effect of *CCNB2* we could not be tested since its target drugs are not available for purchase. In summary, *BUB1B, RRM2* and *ASF1B* were confirmed as druggable targets and drugs (i.e. TG-101209, NVP-TAE684, withaferin-a and panobinostat) targeting them have been identified and validated *in vitro.*

**REFERENCES**

[1] Motzer RJ, Jonasch E, Boyle S, et al. NCCN Guidelines Insights: Kidney Cancer, Version 1.2021. J Natl Compr Canc Netw 2020; 18(9): 1160-70.

[2] National Comprehensive Cancer Network (NCCN). NCCN Clinical Practice Guidelines in Oncology. Kidney Cancer Version 1.2022. 2021. https://www.nccn.org/professionals/physician_gls/pdf/kidney.pdf (accessed July 1, 2021).

[3] Hsieh JJ, Purdue MP, Signoretti S, et al. Renal cell carcinoma. Nat Rev Dis Primers 2017; 3: 17009.

[4] Luo H, Li M, Yang M, Wu FX, Li Y, Wang J. Biomedical data and computational models for drug repositioning: a comprehensive review. Brief Bioinform 2021; 22(2): 1604-19.

[5] Turanli B, Altay O, Boren J, et al. Systems biology based drug repositioning for development of cancer therapy. Semin Cancer Biol 2021; 68: 47-58.

[6] Altay O, Mohammadi E, Lam S, et al. Current Status of COVID-19 Therapies and Drug Repositioning Applications. iScience 2020; 23(7): 101303.

[7] Nagaraj AB, Wang QQ, Joseph P, et al. Using a novel computational drug-repositioning approach (DrugPredict) to rapidly identify potent drug candidates for cancer treatment. Oncogene 2018; 37(3): 403-14.

[8] Xuan P, Cao Y, Zhang T, Wang X, Pan S, Shen T. Drug repositioning through integration of prior knowledge and projections of drugs and diseases. Bioinformatics 2019; 35(20): 4108-19.

[9] Yang M, Wu G, Zhao Q, Li Y, Wang J. Computational drug repositioning based on multi-similarities bilinear matrix factorization. Brief Bioinform 2020.

[10] Zerbini LF, Bhasin MK, de Vasconcellos JF, et al. Computational repositioning and preclinical validation of pentamidine for renal cell cancer. Mol Cancer Ther 2014; 13(7): 1929-41.

[11] Koudijs KKM, Terwisscha van Scheltinga AGT, Bohringer S, Schimmel KJM, Guchelaar HJ. Personalised drug repositioning for Clear Cell Renal Cell Carcinoma using gene expression. Sci Rep 2018; 8(1): 5250.

[12] Lamb J, Crawford ED, Peck D, et al. The Connectivity Map: using gene-expression signatures to connect small molecules, genes, and disease. Science 2006; 313(5795): 1929-35.

[13] Stathias V, Turner J, Koleti A, et al. LINCS Data Portal 2.0: next generation access point for perturbation-response signatures. Nucleic Acids Res 2020; 48(D1): D431-D9.

[14] Li X, Kim W, Juszczak K, et al. Stratification of patients with clear cell renal cell carcinoma to facilitate drug repositioning. iScience 2021; 24(7): 102722.

[15] Pornputtapong N, Nookaew I, Nielsen J. Human metabolic atlas: an online resource for human metabolism. Database (Oxford) 2015; 2015: bavo68.

[16] Mardinoglu A, Boren J, Smith U, Uhlen M, Nielsen J. Systems biology in hepatology: approaches and applications. Nat Rev Gastroenterol Hepatol 2018; 15(6): 365-77.

[17] Hanahan D, Weinberg RA. Hallmarks of cancer: the next generation. Cell 2011; 144(5): 646-74.

[18] Uhlen M, Zhang C, Lee S, et al. A pathology atlas of the human cancer transcriptome. Science 2017; 357(6352).

[19] Li X, Kim W, Arif M, et al. Discovery of Functional Alternatively Spliced PKM Transcripts in Human Cancers. Cancers (Basel) 2021; 13(2).

[20] Han JD. Understanding biological functions through molecular networks. Cell Res 2008; 18(2): 224-37.

[21] Stuart JM, Segal E, Koller D, Kim SK. A gene-coexpression network for global discovery of conserved genetic modules. Science 2003; 302(5643): 249-55.

[22] Pons P, Latapy M. Computing Communities in Large Networks Using Random Walks. Computer and Information Sciences - ISCIS 2005. Berlin, Heidelberg: Springer; 2005.

[23] Wu J, Xia X, Hu Y, Fang X, Orsulic S. Identification of Infertility-Associated Topologically Important Genes Using Weighted Co-expression Network Analysis. Front Genet 2021; 12: 580190.

[24] Bidkhori G, Benfeitas R, Klevstig M, et al. Metabolic network-based stratification of hepatocellular carcinoma reveals three distinct tumor subtypes. Proc Natl Acad Sci USA 2018; 115(50): E11874-E83.

[25] Palmer D, Fabris F, Doherty A, Freitas AA, de Magalhaes JP. Ageing transcriptome meta-analysis reveals similarities and differences between key mammalian tissues. Aging (Albany NY) 2021; 13(3): 3313-41.

[26] Bozhilova LV, Pardo-Diaz J, Reinert G, Deane CM. COGENT: evaluating the consistency of gene co-expression networks. Bioinformatics 2021; 37(13): 1928-9.

[27] Uhlen M, Fagerberg L, Hallstrom BM, et al. Proteomics. Tissue-based map of the human proteome. Science 2015; 347(6220): 1260419.

[28] Uhlen M, Bjorling E, Agaton C, et al. A human protein atlas for normal and cancer tissues based on antibody proteomics. Mol Cell Proteomics 2005; 4(12): 1920-32.

[29] Meyers RM, Bryan JG, McFarland JM, et al. Computational correction of copy number effect improves specificity of CRISPR-Cas9 essentiality screens in cancer cells. Nat Genet 2017; 49(12): 1779-84.

[30] Subramanian A, Narayan R, Corsello SM, et al. A Next Generation Connectivity Map: L1000 Platform and the First 1,000,000 Profiles. Cell 2017; 171(6): 1437-52 e17.

[31] Perlman ZE, Slack MD, Feng Y, Mitchison TJ, Wu LF, Altschuler SJ. Multidimensional drug profiling by automated microscopy. Science 2004; 306(5699): 1194-8.

[32] Cancer Genome Atlas Research N. Comprehensive molecular characterization of clear cell renal cell carcinoma. Nature 2013; 499(7456): 43-9.

[33] Brannon AR, Reddy A, Seiler M, et al. Molecular Stratification of Clear Cell Renal Cell Carcinoma by Consensus Clustering Reveals Distinct Subtypes and Survival Patterns. Genes Cancer 2010; 1(2): 152-63.

[34] Davenport JW, Fernandes ER, Harris LD, Neale GA, Goorha R. The mouse mitotic checkpoint gene bubib, a novel bubi family member, is expressed in a cell cycle-dependent manner. Genomics 1999; 55(1): 113-7.

[35] Whitfield ML, Sherlock G, Saldanha AJ, et al. Identification of genes periodically expressed in the human cell cycle and their expression in tumors. Mol Biol Cell 2002; 13(6): 1977-2000.

[36] Grolmusz VK, Karaszi K, Micsik T, et al. Cell cycle dependent RRM2 may serve as proliferation marker and pharmaceutical target in adrenocortical cancer. Am J Cancer Res 2016; 6(9): 2041-53.

[37] Zhang K, Hu S, Wu J, et al. Overexpression of RRM2 decreases thrombspondin-1 and increases VEGF production in human cancer cells in vitro and in vivo: implication of RRM2 in angiogenesis. Mol Cancer 2009; 8: 11.

[38] Draetta G, Luca F, Westendorf J, Brizuela L, Ruderman J, Beach D. Cdc2 protein kinase is complexed with both cyclin A and B: evidence for proteolytic inactivation of MPF. Cell 1989; 56(5): 829-38.

[39] Petri ET, Errico A, Escobedo L, Hunt T, Basavappa R. The crystal structure of human cyclin B. Cell Cycle 2007; 6(11): 1342-9.

[40] Wu T, Zhang X, Huang X, Yang Y, Hua X. Regulation of cyclin B2 expression and cell cycle G2/m transition by menin. J Biol Chem 2010; 285(24): 18291-300.

[41] Corpet A, De Koning L, Toedling J, et al. Asfib, the necessary Asf1 isoform for proliferation, is predictive of outcome in breast cancer. EMBO J 2011; 30(3): 480-93.

[42] Sekino Y, Han X, Kobayashi G, et al. BUB1B Overexpression Is an Independent Prognostic Marker and Associated with CD44, p53, and PD-L1 in Renal Cell Carcinoma. Oncology 2021; 99(4): 240-50.

[43] Osako Y, Yoshino H, Sakaguchi T, et al. Potential tumorsuppressive role of microRNA99a3p in sunitinibresistant renal cell carcinoma cells through the regulation of RRM2. Int J Oncol 2019; 54(5): 1759-70.

[44] Jiangqiao Z, Tao Q, Zhongbao C, et al. Antisilencing function 1B histone chaperone promotes cell proliferation and migration via activation of the AKT pathway in clear cell renal cell carcinoma. Biochem Biophys Res Commun 2019; 511(1): 165-72.

[45] Peng R, Wang Y, Mao L, Fang F, Guan H. Identification of Core Genes Involved in the Metastasis of Clear Cell Renal Cell Carcinoma. Cancer Manag Res 2020; 12: 13437-49.

[46] Pardanani A, Hood J, Lasho T, et al. TG101209, a small molecule JAK2-selective kinase inhibitor potently inhibits myeloproliferative disorder-associated JAK2V617F and MPLW515L/K mutations. Leukemia 2007; 21(8): 1658-68.

[47] Ramakrishnan V, Kimlinger T, Haug J, et al. TG101209, a novel JAK2 inhibitor, has significant in vitro activity in multiple myeloma and displays preferential cytotoxicity for CD45+ myeloma cells. Am J Hematol 2010; 85(9): 675-86.

[48] Sun Y, Moretti L, Giacalone NJ, et al. Inhibition of JAK2 signaling by TG101209 enhances radiotherapy in lung cancer models. J Thorac Oncol 2011; 6(4): 699-706.

[49] Galkin AV, Melnick JS, Kim S, et al. Identification of NVP-TAE684, a potent, selective, and efficacious inhibitor of NPM-ALK. Proc Natl Acad Sci USA 2007; 104(1): 270-5.

[50] Duong HQ, Than VT, Nguyen HT, et al. Anaplastic lymphoma kinase inhibitor NVPTAE684 suppresses the proliferation of human pancreatic adenocarcinoma cells. Oncol Rep 2021; 45(4).

[51] Schonherr C, Ruuth K, Kamaraj S, et al. Anaplastic Lymphoma Kinase (ALK) regulates initiation of transcription of MYCN in neuroblastoma cells. Oncogene 2012; 31(50): 5193-200.

[52] Ye S, Zhang J, Shen J, et al. NVP-TAE684 reverses multidrug resistance (MDR) in human osteosarcoma by inhibiting P-glycoprotein (PGP1) function. Br J Pharmacol 2016; 173(3): 613-26.

[53] Mohan R, Hammers HJ, Bargagna-Mohan P, et al. Withaferin A is a potent inhibitor of angiogenesis. Angiogenesis 2004; 7(2): 115-22.

[54] Um HJ, Min KJ, Kim DE, Kwon TK. Withaferin A inhibits JAK/STAT3 signaling and induces apoptosis of human renal carcinoma Caki cells. Biochem Biophys Res Commun 2012; 427(1): 24-9.

[55] Choi BY, Kim BW. Withaferin-A Inhibits Colon Cancer Cell Growth by Blocking STAT3 Transcriptional Activity. J Cancer Prev 2015; 20(3): 185-92.

[56] Lee J, Hahm ER, Singh SV. Withaferin A inhibits activation of signal transducer and activator of transcription 3 in human breast cancer cells. Carcinogenesis 2010; 31(11): 1991-8.

[57] Yco LP, Mocz G, Opoku-Ansah J, Bachmann AS. Withaferin A Inhibits STAT3 and Induces Tumor Cell Death in Neuroblastoma and Multiple Myeloma. Biochem Insights 2014; 7: 1-13.

[58] Laubach JP, Moreau P, San-Miguel JF, Richardson PG. Panobinostat for the Treatment of Multiple Myeloma. Clin Cancer Res 2015; 21(21): 4767-73.

[59] Cha TL, Chuang MJ, Wu ST, et al. Dual degradation of aurora A and B kinases by the histone deacetylase inhibitor LBH589 induces G2-M arrest and apoptosis of renal cancer cells. Clin Cancer Res 2009; 15(3): 840-50.

[60] Hainsworth JD, Infante JR, Spigel DR, Arrowsmith ER, Boccia RV, Burris HA. A phase II trial of panobinostat, a histone deacetylase inhibitor, in the treatment of patients with refractory metastatic renal cell carcinoma. Cancer Invest 2011; 29(7): 451-5.

[61] Rausch M, Weiss A, Zoetemelk M, et al. Optimized Combination of HDACI and TKI Efficiently Inhibits Metabolic Activity in Renal Cell Carcinoma and Overcomes Sunitinib Resistance. Cancers (Basel) 2020; 12(11).

[62] Sato A, Asano T, Isono M, Ito K, Asano T. Panobinostat synergizes with bortezomib to induce endoplasmic reticulum stress and ubiquitinated protein accumulation in renal cancer cells. BMC Urol 2014; 14:71.

[63] Tatlow PJ, Piccolo SR. A cloud-based workflow to quantify transcript-expression levels in public cancer compendia. Sci Rep 2016; 6: 39259.

[64] Bray NL, Pimentel H, Melsted P, Pachter L. Near-optimal probabilistic RNA-seq quantification. Nat Biotechnol 2016; 34(5): 525-7.

[65] Colaprico A, Silva TC, Olsen C, et al. TCGAbiolinks: an R/Bioconductor package for integrative analysis of TCGA data. Nucleic Acids Res 2016; 44(8): e71.

[66] Sato Y, Yoshizato T, Shiraishi Y, et al. Integrated molecular analysis of clear-cell renal cell carcinoma. Nat Genet 2013; 45(8): 860-7.

[67] Quinlan AR, Hall IM. BEDTools: a flexible suite of utilities for comparing genomic features. Bioinformatics 2010; 26(6): 841-2.

[68] Ghandi M, Huang FW, Jane-Valbuena J, et al. Next-generation characterization of the Cancer Cell Line Encyclopedia. Nature 2019; 569(7757): 503-8.

[69] Yu G, Wang LG, Han Y, He QY. clusterProfiler: an R package for comparing biological themes among gene clusters. OMICS 2012; 16(5): 284-7.

[70] Csardi G, Nepusz T. The igraph software package for complex network research. InterJournal 2006; Complex Systems: 1695.

[71] Love MI, Huber W, Anders S. Moderated estimation of fold change and dispersion for RNA-seq data with DESeq2. Genome Biol 2014; 15(12): 550.

## Claims

1. A compound or a pharmaceutically acceptable salt or prodrug thereof for use in a method of treatment of a renal cell carcinoma, such as clear cell renal cell carcinoma (ccRCC), wherein the compound is selected from the group consisting of TG-101209, panobinostat, NVP-TAE684 and withaferin-a.

2. The compound for use according to claim 1, wherein the compound is TG-101209.

3. The compound for use according to claim 2, wherein the method of treatment further comprises administration of panobinostat.

4. The compound for use according to claim 2 or 3, wherein the method of treatment further comprises administration of NVP-TAE684 or withaferin-a.

5. The compound for use according to claim 1, wherein the compound is panobinostat.

6. The compound for use according to claim 5, wherein the renal cell carcinoma is stage I-III ccRCC, preferably stage I or II ccRCC.

7. The compound for use according to claim 5 or 6, wherein the method of treatment further comprises administration of TG-101209.

8. The compound for use according to any one of claims 5-7, wherein the method of treatment further comprises administration of NVP-TAE684 or withaferin-a.

9. The compound for use according to claim 1, wherein the compound is NVP- TAE684.

10. The compound for use according to claim 9, wherein the method of treatment further comprises administration of panobinostat.

11. The compound for use according to claim 9 or 10, wherein the method of treatment further comprises administration of TG-101209.

12. The compound for use according to claim 1, wherein the compound is withaferin-a.

13. The compound for use according to claim 12, wherein the method of treatment further comprises administration of panobinostat.

14. The compound for use according to claim 12 or 13, wherein the method of treatment further comprises administration of TG-101209.

15. The compound for use according to any one of the preceding claims, wherein the method of treatment further comprises administration of a drug selected from the group consisting of tyrosine kinase inhibitors (e.g. axitinib, sorafenib, pazopanib and sunitinib), mTOR inhibitors (e.g. everolimus and tesirolimus) and monoclonal antibodies against VEGF (e.g. bevacizumab).

Fig. 1A

Fig. 1B

C

TCGA cohort

Japanese cohort

Fig. 1C

Fig. 1D

Fig. 2A

Fig. 2B

EP 4 238 564 A1

Fig. 2D

Fig. 2C

Fig. 2E

Fig. 2F

Fig. 3

Fig. 4A-C

Fig. 4D-F

Fig. 4G

Fig. 4H

Europäisches
Patentamt

European
Patent Office

Office européen
des brevets

## EUROPEAN SEARCH REPORT

**Application Number**

EP 22 15 9838

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHA TAI-LUNG ET AL: "Dual Degradation of Aurora A and B Kinases by the Histone Deacetylase Inhibitor LBH589 Induces G2-M Arrest and Apoptosis of Renal Cancer Cells", CLINICAL CANCER RESEARCH, vol. 15, no. 3, 1 February 2009 (2009-02-01), pages 840-850, XP055944478, US ISSN: 1078-0432, DOI: 10.1158/1078-0432.CCR-08-1918 Retrieved from the Internet: URL:https://watermark.silverchair.com/840. pdf?token=AQECAHi208BE49Ooan9kkhW_Ercy7Dm3 ZL_9Cf3qfKAc485ysgAAAt8wggLbBgkqhkiG9w0BBw agggLMMIICyAIBADCCAsEGCSqGSIb3DQEHATAeBglg hkgBZQMEAS4wEQQMyJq_aJ4MIjsJvF5rAgEQgIICkq Q5k7qaehnaYasUhtvIM0q7NVUt9zt-dyV3kVIhRr66 i1abil6TbIt-2GDeoX7Tle3YNac6EifXalpbjSaPHb c7u5an5htb> [retrieved on 2022-07-20] | 1,5,6 | INV. A61K31/4045 A61K31/506 A61K31/585 A61P35/00 |
| Y | * the whole document * | 8,13 | |
| | ----- | | **TECHNICAL FIELDS SEARCHED (IPC)** A61K A61P |
| X | OKUBO KAZUKI ET AL: "Panobinostat and Nelfinavir Inhibit Renal Cancer Growth by Inducing Endoplasmic Reticulum Stress", ANTICANCER RESEARCH, vol. 38, no. 10, 1 October 2018 (2018-10-01), pages 5615-5626, XP055944761, GR ISSN: 0250-7005, DOI: 10.21873/anticanres.12896 Retrieved from the Internet: URL:https://ar.iiarjournals.org/content/an ticanres/38/10/5615.full.pdf> | 1,5,6 | |
| Y | * the whole document * | 8,13 | |
| | ----- | | |
| | -/-- | | |

The present search report has been drawn up for all claims

| Place of search | Date of completion of the search | Examiner |
|---|---|---|
| The Hague | 22 July 2022 | Matos de Brito, P |

EPO FORM 1503 03.82 (P04C01)

EUROPÄISCHES Patentamt European Patent Office Office européen des brevets

## EUROPEAN SEARCH REPORT

**Application Number**

EP 22 15 9838

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHOI MIN JUNG ET AL: "Endoplasmic reticulum stress mediates withaferin A-induced apoptosis in human renal carcinoma cells", TOXICOLOGY IN VITRO., vol. 25, no. 3, 1 April 2011 (2011-04-01), pages 692-698, XP055944839, GB ISSN: 0887-2333, DOI: 10.1016/j.tiv.2011.01.010 | 1,12 | |
| Y | * the whole document * | 8,13 | |
| X | Nct01005797: "Study of Panobinostat in Combination With Sorafenib in Kidney, Soft Tissue or Lung Cancers", Clinicaltrials.gov, 3 March 2017 (2017-03-03), XP055944617, Retrieved from the Internet: URL:https://clinicaltrials.gov/ct2/show/NCT01005797 [retrieved on 2022-07-20] * the whole document * | 1,15 | |

TECHNICAL FIELDS SEARCHED (IPC)

The present search report has been drawn up for all claims

| Place of search | Date of completion of the search | Examiner |
|---|---|---|
| The Hague | 22 July 2022 | Matos de Brito, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MOTZER RJ ; JONASCH E ; BOYLE S et al.** NCCN Guidelines Insights: Kidney Cancer, Version 1.2021. *J Natl Compr Canc Netw,* 2020, vol. 18 (9), 1160-70
- NCCN Clinical Practice Guidelines in Oncology. *Kidney Cancer Version 1.2022,* 01 July 2021, https://www.nccn.org/professionals/physician_gls/pdf/kidney.pdf
- **HSIEH JJ ; PURDUE MP ; SIGNORETTI S et al.** Renal cell carcinoma. *Nat Rev Dis Primers,* 2017, vol. 3, 17009
- **LUO H ; LI M ; YANG M ; WU FX ; LI Y ; WANG J.** Biomedical data and computational models for drug repositioning: a comprehensive review. *Brief Bioinform,* 2021, vol. 22 (2), 1604-19
- **TURANLI B ; ALTAY O ; BOREN J et al.** Systems biology based drug repositioning for development of cancer therapy. *Semin Cancer Biol,* 2021, vol. 68, 47-58
- **ALTAY O ; MOHAMMADI E ; LAM S et al.** Current Status of COVID-19 Therapies and Drug Repositioning Applications. *iScience,* 2020, vol. 23 (7), 101303
- **NAGARAJ AB ; WANG QQ ; JOSEPH P et al.** Using a novel computational drug-repositioning approach (DrugPredict) to rapidly identify potent drug candidates for cancer treatment. *Oncogene,* 2018, vol. 37 (3), 403-14
- **XUAN P ; CAO Y ; ZHANG T ; WANG X ; PAN S ; SHEN T.** Drug repositioning through integration of prior knowledge and projections of drugs and diseases. *Bioinformatics,* 2019, vol. 35 (20), 4108-19
- **YANG M ; WU G ; ZHAO Q ; LI Y ; WANG J.** Computational drug repositioning based on multi-similarities bilinear matrix factorization. *Brief Bioinform,* 2020
- **ZERBINI LF ; BHASIN MK ; DE VASCONCELLOS JF et al.** Computational repositioning and preclinical validation of pentamidine for renal cell cancer. *Mol Cancer Ther,* 2014, vol. 13 (7), 1929-41
- **KOUDIJS KKM ; TERWISSCHA VAN SCHELTINGA AGT ; BOHRINGER S ; SCHIMMEL KJM ; GUCHELAAR HJ.** Personalised drug repositioning for Clear Cell Renal Cell Carcinoma using gene expression. *Sci Rep,* 2018, vol. 8 (1), 5250
- **LAMB J ; CRAWFORD ED ; PECK D et al.** The Connectivity Map: using gene-expression signatures to connect small molecules, genes, and disease. *Science,* 2006, vol. 313 (5795), 1929-35

- **STATHIAS V ; TURNER J ; KOLETI A et al.** LINCS Data Portal 2.0: next generation access point for perturbation-response signatures. *Nucleic Acids Res,* 2020, vol. 48 (D), D431-D9
- **LI X ; KIM W ; JUSZCZAK K et al.** Stratification of patients with clear cell renal cell carcinoma to facilitate drug repositioning. *iScience,* 2021, vol. 24 (7), 102722
- **PORNPUTTAPONG N ; NOOKAEW I ; NIELSEN J.** Human metabolic atlas: an online resource for human metabolism. *Database (Oxford),* 2015, vol. 2015, 68
- **MARDINOGLU A ; BOREN J ; SMITH U ; UHLEN M ; NIELSEN J.** Systems biology in hepatology: approaches and applications. *Nat Rev Gastroenterol Hepatol,* 2018, vol. 15 (6), 365-77
- **HANAHAN D ; WEINBERG RA.** Hallmarks of cancer: the next generation. *Cell,* 2011, vol. 144 (5), 646-74
- **UHLEN M ; ZHANG C ; LEE S et al.** A pathology atlas of the human cancer transcriptome. *Science,* 2017, vol. 357 (6352
- **LI X ; KIM W ; ARIF M et al.** Discovery of Functional Alternatively Spliced PKM Transcripts in Human Cancers. *Cancers (Basel),* 2021, vol. 13 (2
- **HAN JD.** Understanding biological functions through molecular networks. *Cell Res,* 2008, vol. 18 (2), 224-37
- **STUART JM ; SEGAL E ; KOLLER D ; KIM SK.** A gene-coexpression network for global discovery of conserved genetic modules. *Science,* 2003, vol. 302 (5643), 249-55
- Computing Communities in Large Networks Using Random Walks. **PONS P ; LATAPY M.** Computer and Information Sciences - ISCIS 2005. Springer, 2005
- **WU J ; XIA X ; HU Y ; FANG X ; ORSULIC S.** Identification of Infertility-Associated Topologically Important Genes Using Weighted Co-expression Network Analysis. *Front Genet,* 2021, vol. 12, 580190
- **BIDKHORI G ; BENFEITAS R ; KLEVSTIG M et al.** Metabolic network-based stratification of hepatocellular carcinoma reveals three distinct tumor subtypes. *Proc Natl Acad Sci USA,* 2018, vol. 115 (50), E11874-E83

- **PALMER D ; FABRIS F ; DOHERTY A ; FREITAS AA ; DE MAGALHAES JP.** Ageing transcriptome meta-analysis reveals similarities and differences between key mammalian tissues. *Aging (Albany NY),* 2021, vol. 13 (3), 3313-41
- **BOZHILOVA LV ; PARDO-DIAZ J ; REINERT G ; DEANE CM.** COGENT: evaluating the consistency of gene co-expression networks. *Bioinformatics,* 2021, vol. 37 (13), 1928-9
- **UHLEN M ; FAGERBERG L ; HALLSTROM BM et al.** Proteomics. Tissue-based map of the human proteome. *Science,* 2015, vol. 347 (6220), 1260419
- **UHLEN M ; BJORLING E ; AGATON C et al.** A human protein atlas for normal and cancer tissues based on antibody proteomics. *Mol Cell Proteomics,* 2005, vol. 4 (12), 1920-32
- **MEYERS RM ; BRYAN JG ; MCFARLAND JM et al.** Computational correction of copy number effect improves specificity of CRISPR-Cas9 essentiality screens in cancer cells. *Nat Genet,* 2017, vol. 49 (12), 1779-84
- **SUBRAMANIAN A ; NARAYAN R ; CORSELLO SM et al.** A Next Generation Connectivity Map: L1000 Platform and the First 1,000,000 Profiles. *Cell,* 2017, vol. 171 (6), 1437-52, e17
- **PERLMAN ZE ; SLACK MD ; FENG Y ; MITCHISON TJ ; WU LF ; ALTSCHULER SJ.** Multidimensional drug profiling by automated microscopy. *Science,* 2004, vol. 306 (5699), 1194-8
- Cancer Genome Atlas Research N. Comprehensive molecular characterization of clear cell renal cell carcinoma. *Nature,* 2013, vol. 499 (7456), 43-9
- **BRANNON AR ; REDDY A ; SEILER M et al.** Molecular Stratification of Clear Cell Renal Cell Carcinoma by Consensus Clustering Reveals Distinct Subtypes and Survival Patterns. *Genes Cancer,* 2010, vol. 1 (2), 152-63
- **DAVENPORT JW ; FERNANDES ER ; HARRIS LD ; NEALE GA ; GOORHA R.** The mouse mitotic checkpoint gene bubib, a novel bubi family member, is expressed in a cell cycle-dependent manner. *Genomics,* 1999, vol. 55 (1), 113-7
- **WHITFIELD ML ; SHERLOCK G ; SALDANHA AJ et al.** Identification of genes periodically expressed in the human cell cycle and their expression in tumors. *Mol Biol Cell,* 2002, vol. 13 (6), 1977-2000
- **GROLMUSZ VK ; KARASZI K ; MICSIK T et al.** Cell cycle dependent RRM2 may serve as proliferation marker and pharmaceutical target in adrenocortical cancer. *Am J Cancer Res,* 2016, vol. 6 (9), 2041-53
- **ZHANG K ; HU S ; WU J et al.** Overexpression of RRM2 decreases thrombspondin-1 and increases VEGF production in human cancer cells in vitro and in vivo: implication of RRM2 in angiogenesis. *Mol Cancer,* 2009, vol. 8, 11
- **DRAETTA G ; LUCA F ; WESTENDORF J ; BRIZUELA L ; RUDERMAN J ; BEACH D.** Cdc2 protein kinase is complexed with both cyclin A and B: evidence for proteolytic inactivation of MPF. *Cell,* 1989, vol. 56 (5), 829-38
- **PETRI ET ; ERRICO A ; ESCOBEDO L ; HUNT T ; BASAVAPPA R.** The crystal structure of human cyclin B. *Cell Cycle,* 2007, vol. 6 (11), 1342-9
- **WU T ; ZHANG X ; HUANG X ; YANG Y ; HUA X.** Regulation of cyclin B2 expression and cell cycle G2/m transition by menin. *J Biol Chem,* 2010, vol. 285 (24), 18291-300
- **CORPET A ; DE KONING L ; TOEDLING J et al.** Asfib, the necessary Asf1 isoform for proliferation, is predictive of outcome in breast cancer. *EMBO J,* 2011, vol. 30 (3), 480-93
- **SEKINO Y ; HAN X ; KOBAYASHI G et al.** BUB1B Overexpression Is an Independent Prognostic Marker and Associated with CD44, p53, and PD-L1 in Renal Cell Carcinoma. *Oncology,* 2021, vol. 99 (4), 240-50
- **OSAKO Y ; YOSHINO H ; SAKAGUCHI T et al.** Potential tumorsuppressive role of microRNA99a3p in sunitinibresistant renal cell carcinoma cells through the regulation of RRM2. *Int J Oncol,* 2019, vol. 54 (5), 1759-70
- **JIANGQIAO Z ; TAO Q ; ZHONGBAO C et al.** Anti-silencing function 1B histone chaperone promotes cell proliferation and migration via activation of the AKT pathway in clear cell renal cell carcinoma. *Biochem Biophys Res Commun,* 2019, vol. 511 (1), 165-72
- **PENG R ; WANG Y ; MAO L ; FANG F ; GUAN H.** Identification of Core Genes Involved in the Metastasis of Clear Cell Renal Cell Carcinoma. *Cancer Manag Res,* 2020, vol. 12, 13437-49
- **PARDANANI A ; HOOD J ; LASHO T et al.** TG101209, a small molecule JAK2-selective kinase inhibitor potently inhibits myeloproliferative disorder-associated JAK2V617F and MPLW515L/K mutations. *Leukemia,* 2007, vol. 21 (8), 1658-68
- **RAMAKRISHNAN V ; KIMLINGER T ; HAUG J et al.** TG101209, a novel JAK2 inhibitor, has significant in vitro activity in multiple myeloma and displays preferential cytotoxicity for CD45+ myeloma cells. *Am J Hematol,* 2010, vol. 85 (9), 675-86
- **SUN Y ; MORETTI L ; GIACALONE NJ et al.** Inhibition of JAK2 signaling by TG101209 enhances radiotherapy in lung cancer models. *J Thorac Oncol,* 2011, vol. 6 (4), 699-706
- **GALKIN AV ; MELNICK JS ; KIM S et al.** Identification of NVP-TAE684, a potent, selective, and efficacious inhibitor of NPM-ALK. *Proc Natl Acad Sci USA,* 2007, vol. 104 (1), 270-5
- **DUONG HQ ; THAN VT ; NGUYEN HT et al.** Anaplastic lymphoma kinase inhibitor NVPTAE684 suppresses the proliferation of human pancreatic adenocarcinoma cells. *Oncol Rep,* 2021, vol. 45 (4

- **SCHONHERR C ; RUUTH K ; KAMARAJ S et al.** Anaplastic Lymphoma Kinase (ALK) regulates initiation of transcription of MYCN in neuroblastoma cells. *Oncogene,* 2012, vol. 31 (50), 5193-200
- **YE S ; ZHANG J ; SHEN J et al.** NVP-TAE684 reverses multidrug resistance (MDR) in human osteosarcoma by inhibiting P-glycoprotein (PGP1) function. *Br J Pharmacol,* 2016, vol. 173 (3), 613-26
- **MOHAN R ; HAMMERS HJ ; BARGAGNA-MOHAN P et al.** Withaferin A is a potent inhibitor of angiogenesis. *Angiogenesis,* 2004, vol. 7 (2), 115-22
- **UM HJ ; MIN KJ ; KIM DE ; KWON TK.** Withaferin A inhibits JAK/STAT3 signaling and induces apoptosis of human renal carcinoma Caki cells. *Biochem Biophys Res Commun,* 2012, vol. 427 (1), 24-9
- **CHOI BY ; KIM BW.** Withaferin-A Inhibits Colon Cancer Cell Growth by Blocking STAT3 Transcriptional Activity. *J Cancer Prev,* 2015, vol. 20 (3), 185-92
- **LEE J ; HAHM ER ; SINGH SV.** Withaferin A inhibits activation of signal transducer and activator of transcription 3 in human breast cancer cells. *Carcinogenesis,* 2010, vol. 31 (11), 1991-8
- **YCO LP ; MOCZ G ; OPOKU-ANSAH J ; BACHMANN AS.** Withaferin A Inhibits STAT3 and Induces Tumor Cell Death in Neuroblastoma and Multiple Myeloma. *Biochem Insights,* 2014, vol. 7, 1-13
- **LAUBACH JP ; MOREAU P ; SAN-MIGUEL JF ; RICHARDSON PG.** Panobinostat for the Treatment of Multiple Myeloma. *Clin Cancer Res,* 2015, vol. 21 (21), 4767-73
- **CHA TL ; CHUANG MJ ; WU ST et al.** Dual degradation of aurora A and B kinases by the histone deacetylase inhibitor LBH589 induces G2-M arrest and apoptosis of renal cancer cells. *Clin Cancer Res,* 2009, vol. 15 (3), 840-50
- **HAINSWORTH JD ; INFANTE JR ; SPIGEL DR ; ARROWSMITH ER ; BOCCIA RV ; BURRIS HA.** A phase II trial of panobinostat, a histone deacetylase inhibitor, in the treatment of patients with refractory metastatic renal cell carcinoma. *Cancer Invest,* 2011, vol. 29 (7), 451-5
- **RAUSCH M ; WEISS A ; ZOETEMELK M et al.** Optimized Combination of HDACI and TKI Efficiently Inhibits Metabolic Activity in Renal Cell Carcinoma and Overcomes Sunitinib Resistance. *Cancers (Basel),* 2020, vol. 12 (11
- **SATO A ; ASANO T ; ISONO M ; ITO K ; ASANO T.** Panobinostat synergizes with bortezomib to induce endoplasmic reticulum stress and ubiquitinated protein accumulation in renal cancer cells. *BMC Urol,* 2014, vol. 14, 71
- **TATLOW PJ ; PICCOLO SR.** A cloud-based workflow to quantify transcript-expression levels in public cancer compendia. *Sci Rep,* 2016, vol. 6, 39259
- **BRAY NL ; PIMENTEL H ; MELSTED P ; PACHTER L.** Near-optimal probabilistic RNA-seq quantification. *Nat Biotechnol,* 2016, vol. 34 (5), 525-7
- **COLAPRICO A ; SILVA TC ; OLSEN C et al.** TCGAbiolinks: an R/Bioconductor package for integrative analysis of TCGA data. *Nucleic Acids Res,* 2016, vol. 44 (8), e71
- **SATO Y ; YOSHIZATO T ; SHIRAISHI Y et al.** Integrated molecular analysis of clear-cell renal cell carcinoma. *Nat Genet,* 2013, vol. 45 (8), 860-7
- **QUINLAN AR ; HALL IM.** BEDTools: a flexible suite of utilities for comparing genomic features. *Bioinformatics,* 2010, vol. 26 (6), 841-2
- **GHANDI M ; HUANG FW ; JANE-VALBUENA J et al.** Next-generation characterization of the Cancer Cell Line Encyclopedia. *Nature,* 2019, vol. 569 (7757), 503-8
- **YU G ; WANG LG ; HAN Y ; HE QY.** clusterProfiler: an R package for comparing biological themes among gene clusters. *OMICS,* 2012, vol. 16 (5), 284-7
- **CSARDI G ; NEPUSZ T.** The igraph software package for complex network research. *InterJournal,* 2006, 1695
- **LOVE MI ; HUBER W ; ANDERS S.** Moderated estimation of fold change and dispersion for RNA-seq data with DESeq2. *Genome Biol,* 2014, vol. 15 (12), 550